(19) Europäisches Patentamt
European Patent Office
Office européen des brevets

(11) EP 0 545 774 B1

(12) **FASCICULE DE BREVET EUROPEEN**

(45) Date de publication et mention de la délivrance du brevet: **14.08.1996 Bulletin 1996/33**

(51) Int. Cl.[6]: **B01J 31/02**, C07C 53/42

(21) Numéro de dépôt: **92403160.2**

(22) Date de dépôt: **24.11.1992**

(54) **Supports de silice modifiés par greffage de groupements polyalkylguanidinium, leur procédé d'obtention et application comme catalyseurs**

Mit dem Pfropfen von Polyalkylguanidiniumgruppen modifizierte Silikaträger und Verfahren für ihre Herstellung und ihre Verwendung als Katalysatoren

Silica supports modified by grafting polyalkylguanidinium groups, process for their preparation and their use as catalysts

(84) Etats contractants désignés:
**BE CH DE ES FR GB IT LI NL**

(30) Priorité: **29.11.1991 FR 9114847**

(43) Date de publication de la demande:
**09.06.1993 Bulletin 1993/23**

(73) Titulaire: **SOCIETE NATIONALE DES POUDRES ET EXPLOSIFS**
**F-75181 Paris Cédex 04 (FR)**

(72) Inventeurs:
- **Gauthier, Patricia**
  **F-91590 La Ferte Alais (FR)**
- **Gros, Philippe**
  **F-42000 Saint Etienne (FR)**
- **Le Perchec, Pierre**
  **F-69003 Lyon (FR)**
- **Senet, Jean-Pierre**
  **F-77760 La Chapelle La Reine (FR)**

(74) Mandataire: **Phélip, Bruno et al**
**c/o Cabinet Harlé & Phélip**
**21, rue de La Rochefoucauld**
**75009 Paris (FR)**

(56) Documents cités:
**EP-A- 0 028 107**
**EP-A- 0 285 937**
**EP-A- 0 168 167**
**US-A- 4 613 491**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Rank Xerox (UK) Business Services
2.13.0/3.4

EP 0 545 774 B1

## Description

La présente invention a pour objet des supports à base de silice modifiés en surface par greffage de groupements polyalkylguanidinium.

Elle est d'autre part relative à leur obtention ainsi qu'à leur utilisation comme catalyseurs de chloruration et d'estérification.

La littérature propose de nombreux catalyseurs des réactions de chloruration , notamment pour la préparation de chlorures d'acides carboxyliques par réaction des acides correspondants avec un agent chlorurant tel qu'en particulier le phosgène qui est le plus souvent utilisé .

Malheureusement , tous ces catalyseurs qui sont au moins partiellement solubles dans les milieux organiques , posent deux problèmes majeurs à l'homme du métier :

- pour atteindre une bonne pureté , il est nécessaire de les éliminer du produit fini par distillation. Cette opération toujours coûteuse se révèle difficile, voire impossible dans le cas des chlorures d'acides thermosensibles ou de masses molaires trop élevées .
- pour des raisons économiques , il est souvent indispensable de pouvoir les réutiliser sans recourir à des opérations délicates, ce qui est toujours le cas quand on les récupère dans les culots de distillation. Ces derniers contiennent , en effet , plusieurs autres produits de dégradation souvent gênants .

De plus , le recyclage de ces catalyseurs peut se révéler impossible du fait de leur décomposition .

Deux groupes de catalyseurs de phosgénation sont ainsi particulièrement décrits dans la littérature .

Le premier groupe comprend les catalyseurs de phosgénation à chaud , qui ne sont pas fixés sur support insoluble .

L'art antérieur [ voir notamment J. COTTER et al. Chemistry and Industry 8/5/65 pp.791-3 ] en décrit une grande variété parmi lesquels on peut citer :

- les amines tertiaires et leurs chlorhydrates, (brevet FR 2.212.319),
- les amides N,N disubstitués ( brevet US 3.149.155),
- les sels d'ammonium et de phosphonium quaternaires ( brevet GB 1.159.266 ) .
- les tétraalkylurées ( brevet GB 1.161.220 ) ;

Les catalyseurs récupérés lors des opérations de distillation dans les culots sont généralement mélangés à un grand nombre de produits lourds de dégradation, ce qui rend délicat leur recyclage dans les opérations de phosgénation ultérieures.

Il faut noter aussi que plusieurs types de ces catalyseurs sont instables thermiquement , se décomposent en sous-produits gênants et ne peuvent être récupérés et réutilisés .

Il a d'autre part été montré [ brevet FR 2.585.351 ] que des sels d'hexaalkylguanidinium sont extrêmement efficaces comme catalyseurs de phosgénation des acides carboxyliques et peuvent être mis en oeuvre en très faibles quantités .

Cependant , bien qu'ils marquent un net progrès par rapport aux autres catalyseurs, il est toujours nécessaire de les éliminer par distillation pour obtenir des chlorures d'acide de très grande pureté . Outre le fait que cette opération est coûteuse, elle est parfois impossible lorsque les produits sont trop instables ou lorsque leur point d'ébullition est trop élevé. On est alors contraint de livrer un produit impur contenant du catalyseur résiduel , ce qui n'est pas toujours acceptable , notamment pour des applications pharmaceutiques .

Le second groupe comprend les catalyseurs de phosgénation fixés sur supports insolubles .

Les problèmes exposés précédemment n'ont pas échappé aux fabricants de produits issus de phosgénations à chaud . Ils ont ainsi proposé des catalyseurs chimiquement fixés sur des polymères insolubles dans les milieux organiques, tels que les polymères de structures suivantes :

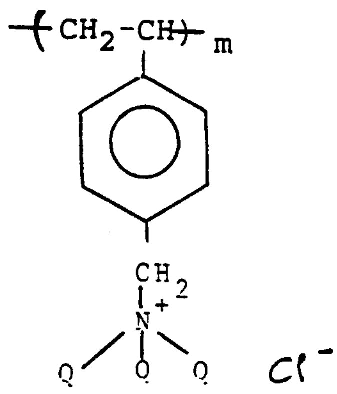

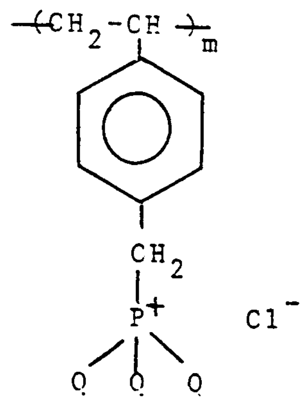

Ces polymères dérivés de polystyrènes chlorométhylés , sont décrits comme catalyseurs de phosgénation:

- des phénols [ brevet US 3.211.775 ]
- des mercaptans [ brevet FR 2.462.423 ]
- des acides carboxyliques [ brevet JP 59. 108 736; Chemical Abstract 101, 151 439 e ].

Ce sont par exemple des résines échangeuses d'anions réticulées avec 7-8% de divinylbenzène, telles que la résine commercialisée sous la dénomination AMBERLITE IRA-400 par ROHM & HAAS.

Des essais effectués par la demanderesse ont cependant montré que ces systèmes catalytiques présentent l'inconvénient d'être détruits dès la première utilisation en raison de la fragilisation benzylique très importante à partir de 80-90°C , ce qui rend impossible le recyclage . On obtient de plus un produit impur .

La fragilisation benzylique peut être représentée schématiquement de la manière suivante dans lequel Bu représente le groupe butyle .

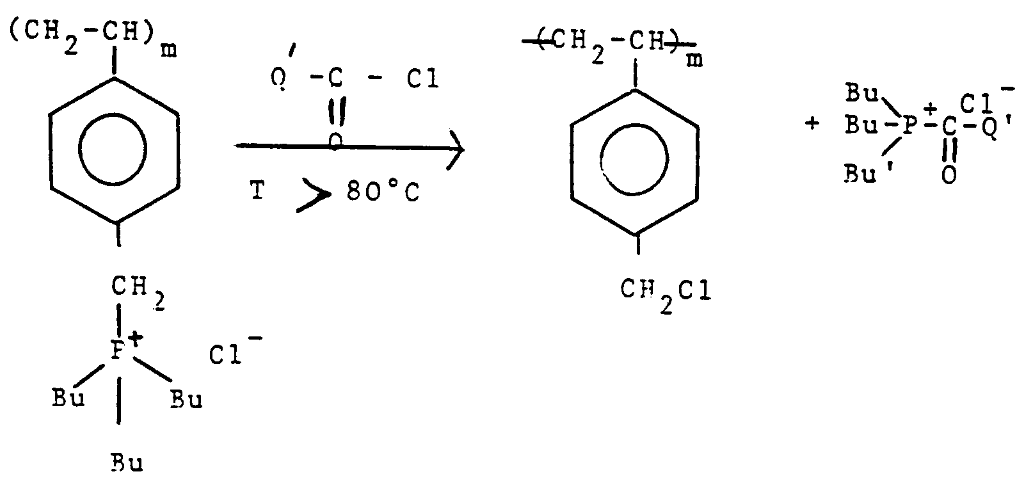


Lorsqu'on utilise un agent chlorurant autre que le phosgène pour obtenir des chlorures d'acides, des catalyseurs sont aussi très souvent nécessaires . Par exemple avec le chlorure de thionyle, les chlorures d'acides forts ne peuvent être obtenus qu'avec un catalyseur du même type que ceux précédemment mentionnés et avec les mêmes inconvénients.

La préparation d'esters par réaction d'acides carboxyliques avec des chloroformiates pose également de nombreux problèmes . Si l'utilisation de catalyseurs tels que la 4-diméthylaminopyridine a permis d'améliorer les rendements de cette réaction, l'obtention sélective d'esters est encore difficile à cause des réactions concurrentes qui produisent des anhydrides et des carbonates, en particulier lorsque les acides contiennent des radicaux stériquement encombrants.

A la connaissance de la demanderesse l'art antérieur ne mentionne pas de catalyseurs efficaces, stables et facilement recyclables, fixés sur des supports à base de silice utilisables dans des réactions de chloruration d'acides carboxyliques ou d'estérification au moyen de chloroformiates.

Le greffage de molécules organiques sur de tels supports est néanmoins décrit dans l'art antérieur (De Haan et al., J. of Colloid. Inter. Sci., 1986, 110, p.591) mais à la connaissance de la demanderesse, le greffage des groupements guanidinium polysubstitués n'a pas été enseigné.

Ainsi, des catalyseurs constitués par des guanidines fixées sur un support de silice ont été décrits dans la demande EP 168.167 mais ces composés sont des bases et de ce fait ne sont utiles que pour effectuer des réactions catalysées par des bases fortes.

La demanderesse a trouvé de manière surprenante que l'on pouvait fixer sur des supports à base de silice des ions guanidinium polysubstitués.

La demanderesse a trouvé de manière encore plus surprenante que de tels supports permettaient de catalyser efficacement les réactions de chloruration et d'estérification et étaient recyclables par des méthodes simples.

La présente invention a donc pour objet des supports à base de silice, modifiés en surface par greffage par liaisons covalentes de groupements organiques caractérisé en ce que lesdits groupements sont des groupements guanidinium polysubstitués, et de préférence des groupements polyalkyl-guanidinium.

La présente invention a plus particulièrement pour objet des supports répondant à la formule (I) suivante :

(I)

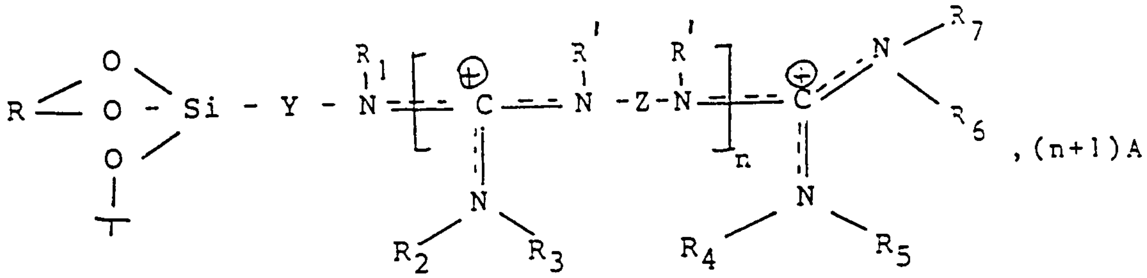

dans laquelle :

- R représente le support à base de silice .
- Y représente un radical alkyle bivalent ramifié ou non de $C_2$ à $C_{10}$ , comportant au moins 2 atomes de carbone sur la chaîne linéaire entre les atomes de silicium et d'azote, de préférence Y étant le radical $-(CH_2)_3-$,
- $R_1$, $R_2$, $R_3$, $R_4$, $R_5$, $R_6$, $R_7$, identiques ou différents , représentent des radicaux alkyles de $C_1$ à $C_6$, de préférence les radicaux méthyle et/ou n-butyle.
- A représente un anion possédant une seule charge négative , de préférence un ion halogénure éventuellement associé à l'acide halohydrique correspondant et en particulier un ion chlorure ou un ion $HCl_2^-$ ,
- Z représente un radical aliphatique bivalent de $C_2$ à $C_{10}$,
- R' représente un radical alkyle de $C_1$ à $C_4$,
- n = 0 ou n est un nombre entier, généralement inférieur à 10, de préférence inférieur à 5 .
- T représente :

  * une liaison simple avec le support R
  * un groupe $-Si(R_o)_3$ dans lequel $R_o$ est un alkyle en $C_1$ à $C_4$, préférentiellement un résidu méthyle, ou
  * un groupe alkyle en $C_1$ à $C_4$

La présente invention est d'autre part relative à l'ion triméthoxysilane-polyalkylguanidinium de formule (II) suivante :

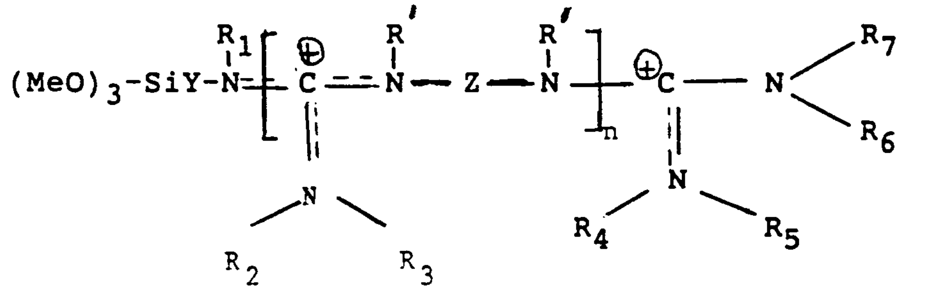

(II)

dans laquelle R', Z , Y, n et $R_1$ à $R_7$ ont les mêmes significations que précédemment et Me représente le radical méthyle .

La présente invention est en outre relative à un procédé de préparation des supports précédemment décrits .

Les supports de formule (I) peuvent être obtenus par réaction du support à base de silice avec un ion polyalkylguanidinium en particulier de formule (II).

Lorsque n = 0 , l'ion de formule (II) peut être lui-même préparé par réaction d'une amine primaire avec un halogénure de triméthoxysilylalkyle puis par réaction de la triméthoxysilylamine secondaire obtenue avec un sel de formamidinium . La première réaction s'effectue généralement avec un excès de l'amine , éventuellement dans un solvant inerte . Pour la seconde réaction, on opère généralement en présence d'une base organique telle que la triéthylamine , dans un solvant inerte. Le schéma réactionnel avec un anion chlorure est le suivant :

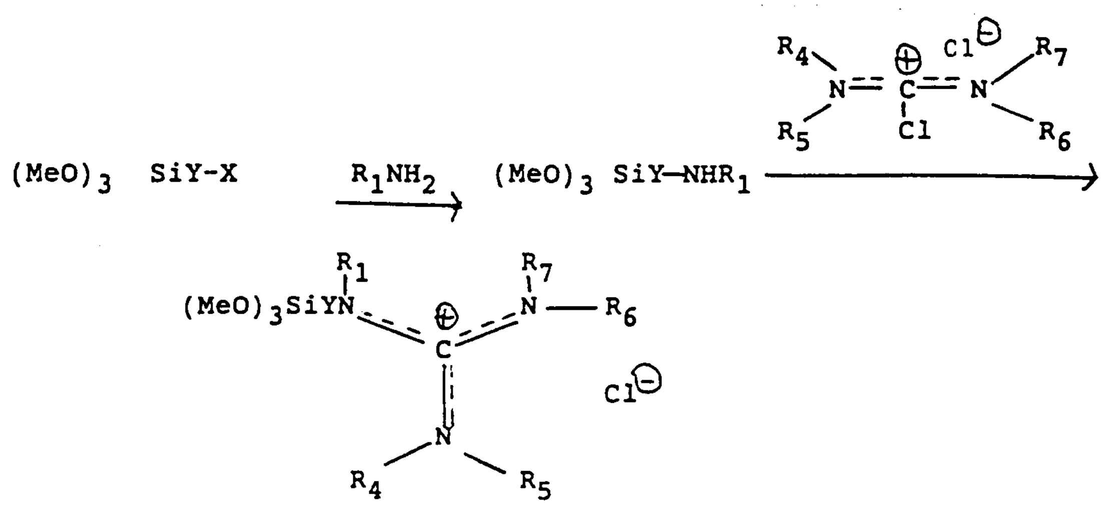


dans lequel X est un halogène,en particulier Cl ou Br.

Lorsque n est différent de O , l'ion de formule (II) peut être obtenu par une première étape de polycondensation d'un sel de diformamidinium avec une diamine pour obtenir un polyguanidinium possédant une terminaison formamidinium. Dans une deuxième étape, le polyguanidinium obtenu précédemment est mis à réagir avec une triméthoxysilylalkylamine pour obtenir un polymère modifié puis dans une troisième étape le polymère modifié est mis à réagir avec un sel de formamidinium.

La première réaction a lieu en général dans un solvant inerte tel qu'un hydrocarbure aliphatique chloré par exemple le dichloroéthane ou le chlorure de méthylène à une température comprise entre 20°C et 100°C et en présence d'une base organique telle qu'une triéthylamine . La deuxième et la troisième étapes s'effectuent dans les mêmes conditions.

Le schéma réactionnel avec un anion chlorure est le suivant . Si un autre anion est préféré, il est possible de changer d'anions en traitant un des intermédiaires par un sel.

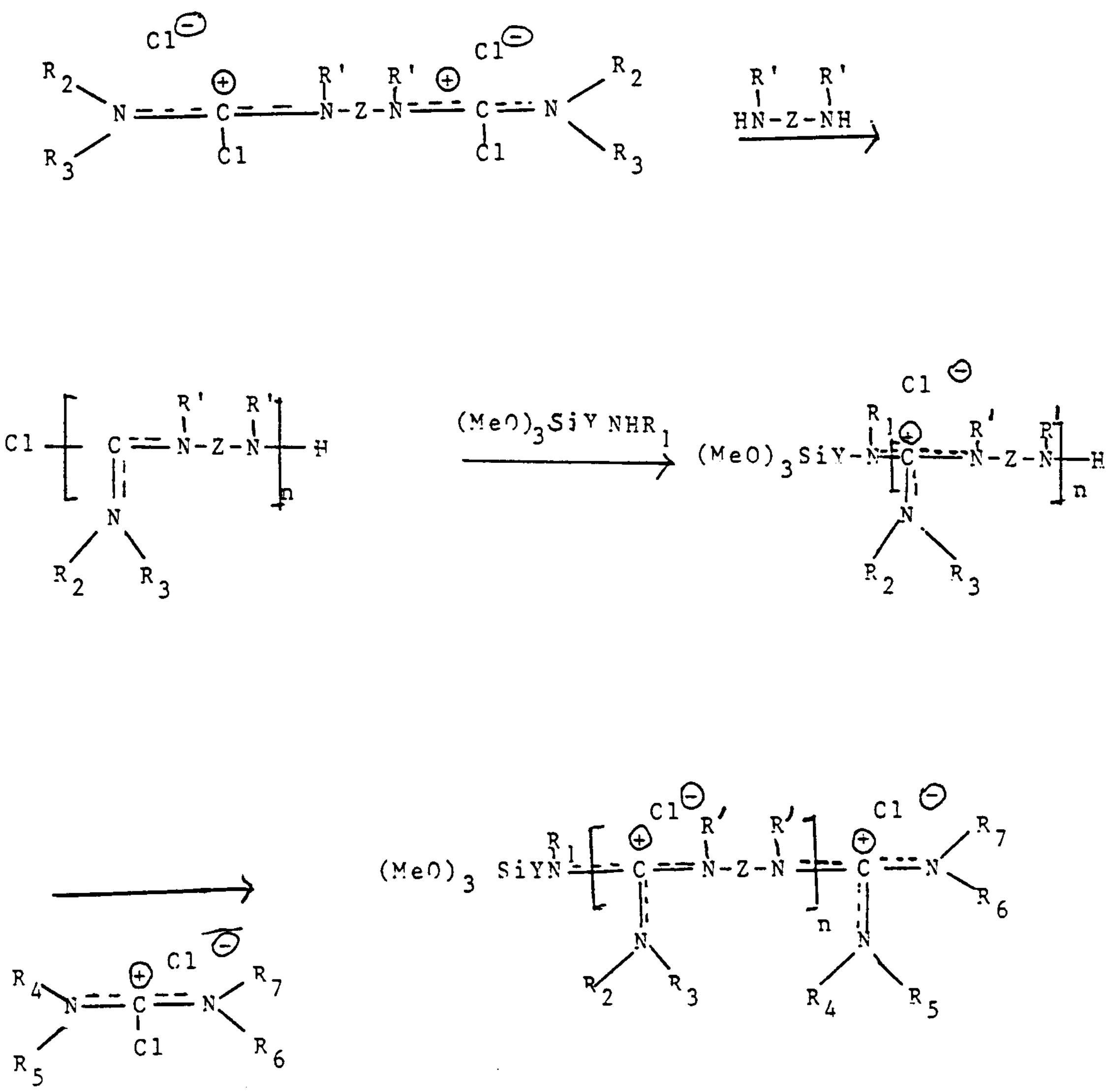

De manière complémentaire , on peut ajouter que les chlorures de polyalkylchloroimmoniums:

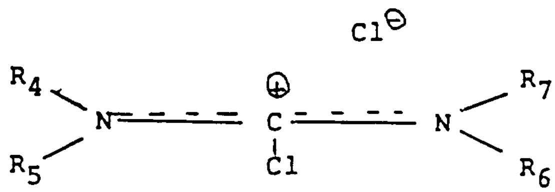

et

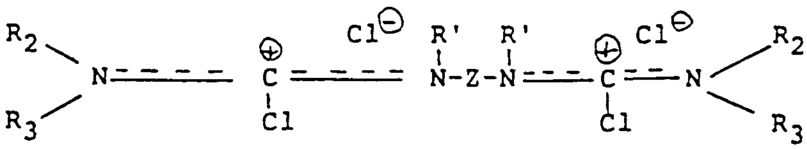

sont préparés de manière connue , par exemple par réaction du phosgène [ H. EILINGSFELD Chem. Ber.(1964) 97, p. 1232 ] ou du chlorure d'oxalyle [ H. ULRICH Angew. Chem.Int. Ed. Engl. (1966) 5 704 ] avec les urées substituées .

Les conditions générales de fonctionnalisation des silices à groupes -OH de surface sont indiquées dans De Haan et al. ( J. of Colloid. Inter. Sci., 1986, 110, p.591).

Enfin, pour améliorer la stabilité des supports, on peut transformer les fonctions silanol résiduelles du support par traitement final de façon connue, par exemple par l'hexaméthyldisilazane selon la réaction :

$$R\text{-}OH + (CH_3)_3SiNHSi(CH_3)_3 \rightarrow R\text{-}OSi\ Me_3$$

Le support à base de silice utilisé est préférentiellement poreux et sous forme de billes dont le diamètre est choisi , pour des raisons de facilité de filtration, entre 0,01 et 1 mm et de préférence entre 0,1 et 1 mm. La surface spécifique est comprise entre 50 et 750 $m^2/g$, de préférence entre 50 et $200 m^2/g$.

La concentration en fonctions silanol de surface est avantageusement de l'ordre de $4{,}8.10^{-6}$ mole/$m^2$ .

Les supports précédemment décrits peuvent être utilisés pour la catalyse de la transformation des acides carboxyliques en chlorures d'acide au moyen d'agents chlorurants tels que par exemple le phosgène, le diphosgène (chloroformiate de trichlorométhyle), le triphosgène ( hexachlorodiméthylcarbonate), le chlorure de thionyle, le chlorure d'oxalyle et le trichlorure de phosphore .

Tous les acides que l'on sait transformer en chlorures d'acide par chloruration avec un agent chlorurant peuvent être utilisés comme matières de départ dans le procédé selon l'invention .

Par exemple , lesdits acides carboxyliques répondent à la formule (III):

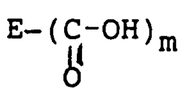

(III)

dans laquelle :

* lorsque m = 1,
  E représente
  - un radical aliphatique linéaire ou ramifié, saturé ou non, substitué ou non, de $C_1$ a $C_{30}$,
  - un radical cycloaliphatique de $C_3$ à $C_7$,
  - un radical aromatique substitué ou non,
  - un radical hétérocyclique
* lorsque m est un nombre entier supérieur à 1 E représente
  - un radical plurivalent linéaire ou ramifié, saturé ou non, substitué ou non de $C_2$ à $C_{20}$
  - un radical cycloaliphatique de $C_5$ à $C_7$,
  - un radical aromatique substitué ou non,

- un radical hétérocyclique.

Les conditions réactionnelles de chloruration des acides carboxyliques au moyen du phosgène, du diphosgène ou du triphosgène en présence de catalyseurs selon l'invention sont indiquées ci-dessous .

Lorsqu'on opère en discontinu , on peut employer des quantités de composés de formule [I] correspondant à des quantités de groupes guanidinium fixés comprises entre $10^{-3}$ et $5.10^{-2}$ eq. par eq. de fonction acide à transformer .

Par exemple , pour un support à base de silice contenant 0,2 meq/g de fonctions guanidinium greffées (détermination par taux de $Cl^{-}$), on utilisera, pour une mole de monoacide , une quantité de catalyseur comprise entre 5g et 250 g.

Lorsqu'on veut travailler en continu, on fait passer l'acide et le phosgène ou un de ses homologues sur un lit de catalyseur, le débit étant choisi de telle sorte que le temps de contact des réactifs soit suffisant .

On préfère , lorsque cela est possible , opérer sans solvant . Dans certains cas, par exemple lorsque les acides ont des points de fusion élevés, supérieurs à 140°C ou quand on met en oeuvre de grandes quantités de silice greffée on peut utiliser un solvant inerte tel que: le toluène, le xylène, le chlorobenzène, les dichlorobenzènes et autres .

La réaction de phosgénation selon l'invention est généralement effectuée à une température comprise entre 80°C et 160°C et de préférence entre 90°C et 130°C.

La durée de la réaction est fonction de la nature de l'acide à transformer, de la quantité de fonctions guanidinium engagée, de la structure du catalyseur et de la température . Elle est généralement comprise entre 2 h et 24 h environ.

Lorsqu'on utilise un agent chlorurant autre que le phosgène ou ses dérivés, on adapte les conditions opératoires en fonction des caractéristiques propres et connues de cet agent chlorurant. Avec le chlorure de thionyle , par exemple , on opère dans les mêmes conditions mais généralement avec une température légèrement inférieure comprise entre 50° et 100°C.

Le procédé de chloruration selon l'invention permet d'obtenir des chlorures d'acide très purs et de recycler intégralement les catalyseurs utilisés .

Il est connu que les chlorures d'acides carboxyliques sont des intermédiaires de synthèse largement utilisés dans de multiples domaines de l'industrie chimique tels que la pharmacie , la phytopharmacie, la chimie des polymères (synthèse de per-esters initiateurs de polymérisation radicalaire par exemple ), la fabrication des produits cosmétiques ou le traitement du papier.

Les supports selon la présente invention peuvent être également utilisés comme catalyseurs pour former des esters par réaction des acides carboxyliques avec des chloroformiates.

Tous les acides précédemment cités peuvent être utilisés comme matière de départ.

Les chloroformiates connus pour former des esters comme par exemple ceux décrits dans la publication de Kim S., Lee J.I., Kim Y.C., dans J. Org. Chem., 1985, 50, 560 peuvent être utilisés. Ce sont en particulier les chloroformiates aliphatiques en $C_2$ à $C_{10}$ , linéaires ou ramifiés, substitués ou non, araliphatiques substitués ou non, aromatiques substitués ou non.

Les quantités de catalyseurs ajoutées sont généralement comprises dans les mêmes proportions que précédemment indiqué pour l'obtention des chlorures d'acide .

On opère généralement sans solvant ou dans un milieu solvant tel que cité précédemment.

La température est généralement comprise entre 80° et 140°C, de préférence entre 110° et 130°C.

Les catalyseurs selon la présente invention sont particulièrement utiles pour former des esters à partir des acides possédant des radicaux stériquement encombrants. Les esters sont obtenus sélectivement avec des rendements très élevés et avec une grande pureté. Il ne se forme pratiquement pas en même temps des anhydrides ou des carbonates comme dans les procédés antérieurs utilisant d'autres catalyseurs. Les catalyseurs selon l'invention ne perdent pas leur propriété et sont facilement recyclables.

Les esters sont des intermédiaires de synthèses très utilisés notamment pour la protection des fonctions acides ou pour le couplage des acides aminés en synthèse peptidique.

La présente invention est illustrée sans pour autant être limitée par les exemples d'application suivants :

EXEMPLE 1 :

Préparation d'un support à base de silice de formule [ I] dans laquelle n = 0 .

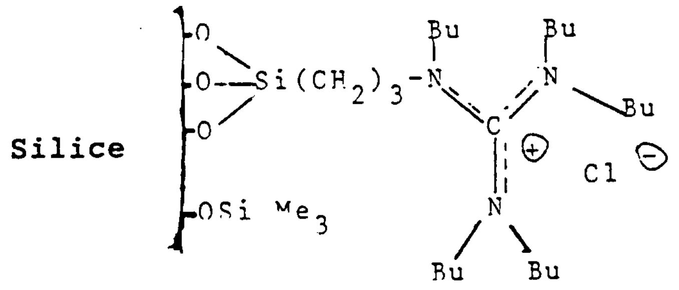

ou

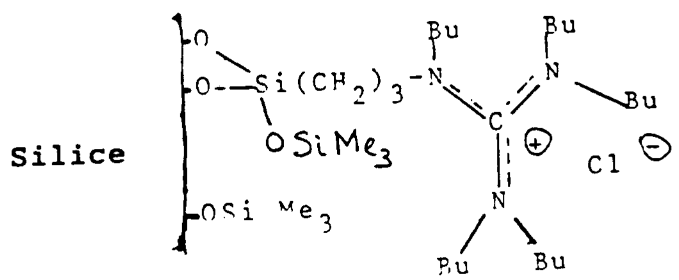

Etape 1 . Synthèse du N-butylamino-(propyltriméthoxysilane).

22g (0,3 mole) de n-butylamine distillée sont portés au reflux (80°C). 18,6 ml (0,1 mole) de (3-chloropropyl)-triméthoxysilane sont alors additionnés lentement sous courant d'azote . Le mélange est laissé au reflux de la n-butylamine pendant 12 heures.

Après refroidissement , on obtient un précipité abondant de chlorhydrate de n-butylamine dont la filtration est facilitée par addition de 100 ml d'éther de pétrole (45-60°C).

Le filtrat est alors concentré et le liquide jaune obtenu est ensuite purifié par distillation à 80°C sous une pression de 13,3 Pa ( 0,1 mm Hg) .

On obtient alors 21 g (rendement 88%) de liquide incolore qui doit être stocké à l'abri de la lumière .

Analyses.

RMN 80 MHz:δppm : 0,6-0,9 (m ; 5H ; $CH_3$-$CH_2$-;$CH_2$-Si-); 1,2-1,8 (m; 7H; -$CH_2$-; -N-H); 2,5 (q; 4H; -$CH_2$-NH-); -3,4 (s ; 9H; $CH_3$O-).

| Analyse élémentaire : | | | | | |
|---|---|---|---|---|---|
| | %C | %H | %N | %O | %Si |
| Calculé | 51,02 | 10,71 | 5,95 | 20,39 | 11,93 |
| Trouvé | 50,83 | 10,42 | 5,70 | * | 12,25 |
| * Le taux d'oxygène ne peut être mesuré en présence de silicium. | | | | | |

Etape 2. Synthèse du chlorure de (N,N, N',N',N"-pentabutyl, N" propyl triméthoxysilane guanidinium.

A une solution de 10g ( 0,042 mole) du silane précédent et de 8,3 ml (0,06 mole) de triéthylamine dans 30 ml de 1,2 dichloroéthane sec , on ajoute lentement sous courant d'azote une solution de 14,2 g ( 0,042 mole) de chlorure de tétrabutyl-chloroamidinium (TBCA) dans 20 ml de 1,2 dichloroéthane en 2 heures.

La réaction est légèrement exothermique et la précipitation du chlorhydrate de triéthylamine est immédiate .

Le milieu réactionnel est agité à température ambiante pendant 24 heures.

Après séparation des chlorhydrates, le filtrat est concentré et repris par 20 ml d'éther de pétrole (45-60°C). La phase visqueuse marron obtenue est séparée et séchée à l'évaporateur rotatif pendant 3 heures à 80°C sous une pression de 2 kPa (15 mm Hg). On obtient ainsi 16 g (71%) d'un liquide visqueux marron.

Analyses.

IRTF : bande du guanidinium $1540 cm^{-1}$

RMN 80 MHz: δppm : 0,6-1 (m; 17H; $CH_3$-$CH_2$-; $CH_2$-Si-); 1,1-1,9 (m; 22H; -$CH_2$-$CH_2$-); 2,9-3,2 (m; 12H; -$CH_2$-N-$C^+$-) ; 3,6 (s ; 9H; $CH_3O$-).

| Analyse élémentaire . | | | | | | |
|---|---|---|---|---|---|---|
| | % C | %H | %N | %O | %Cl | % Si |
| Calculé | 60,24 | 11,23 | 7,81 | 8,92 | 6,59 | 5,22 |
| Trouvé | 60,00 | 10,92 | 7,51 | * | 6,79 | 5,35 |
| * voir analyse de l'étape 1. | | | | | | |

Etape 3. Greffage du silane obtenu dans l'étape 2 sur silice.

A 10 g de silice (90 $m^2$/g ,4,8 µmol/$m^2$) dans 50 ml de toluène sec on ajoute une solution de 3,4 g ($6,3.10^{-3}$ moles) de silane de l'étape 2 dans 50 ml de toluène sec sous courant d'azote .

Le mélange est porté au reflux pendant 24 heures, la solution est alors filtrée et la silice lavée par deux fois 50 ml de 1,2-dichloroéthane sec.

Etape 4. Blocage des fonctions silanols résiduelles .

La silice obtenue précédemment est traitée par une solution de 2,6 ml (0,012 mole) d'hexaméthyldisilazane (HMDS) dans 50 ml de tétrachlorure de carbone pendant 2 heures à température ambiante .

La silice est alors filtrée et lavée pendant 4 heures au soxhlet avec du dichlorométhane puis séchée pendant 12 heures à 60°C sous 2,6 kPa ( 20 mm Hg ) .

Analyses :

IRTF (réflexion diffuse): bande guanidinium 1540 $cm^{-1}$ bandes -C-H à 2980-2990$cm^{-1}$

| Analyse élémentaire : | | | | | |
|---|---|---|---|---|---|
| | C% | H% | N% | Si% | Cl% |
| Trouvé | 4,37 | 1,14 | 0,65 | 43,40 | 0,60 |

Taux de chlore : 0,2 meq/g.

EXEMPLE 2 :

Préparation d'un support à base de silice de formule [I] dans laquelle n = 3 ,Y = $(CH_2)_3$- ,R' = Me; $R_1$=$R_2$=$R_3$=$R_4$=$R_5$=$R_6$=$R_7$= n-Bu, Z=-$(CH_2)_6$-.

Etape 1 : Synthèse de la di N,N'-(dibutylaminocarbonyl) N,N'-diméthyl-hexane-diamine-1,6.

A une solution de 4,33 g (0,03 mole) de N,N'-diméthyl-hexane-diamine-1,6 et 10 ml (0,07 mole) de triéthylamine dans 100 ml de benzène anhydre, on ajoute rapidement sous atmosphère inerte une solution de 11,5 g (0,06 mole) de chlorure de N,N-dibutyl carbamoyle dans 50 ml de benzène anhydre, et on chauffe à reflux pendant 24 heures . Après refroidissement , on lave par 4 fois avec 100 ml d'eau, on sèche sur du sulfate de sodium , on concentre à sec puis purifie par distillation au four à boules (P = 1,33 Pa ( 0,01 mm Hg) ; T° du four = 200°C.).

On obtient 8,2 g (60%) d'huile jaune pâle.

I.R.T.F. : Urée : bande à 1647 $cm^{-1}$ ; disparition de la bande carbamoyle à 1732 $cm^{-1}$.

R.M.N. $^1$H 80 MHz (CDCl3; TMS) δ ppm: 0,7 (m,12 H); 1,1-1,7 (m,24H); 2,8 (s,6H) 3,1 (t,J=7Hz, 12H).

Etape 2.

Synthèse du dichlorure de di (N',N'-dibutyl N-méthyl-chloroformamidinium)-1,6 hexane.

A une solution de 7g (0,0154 mole) de la diurée obtenue dans l'étape 1 dans 20 ml de dichloroéthane sec, on ajoute une solution de 4 ml (0,046 mole) de chlorure d'oxalyle dans 10 ml de dichloroéthane, sous atmosphère sèche, puis on laisse réagir pendant 18 heures à 70°C ( on suit l'avancement de la réaction par spectrométrie infrarouge et l'on procède éventuellement à l'addition d'une nouvelle quantité de chlorure d'oxalyle (1 ml) suivie d'un nouveau temps de réaction de 4 heures ) . Quand la bande urée à 1647 $cm^{-1}$ a complètement disparu, le spectre infra-rouge (I.R.) présente une bande unique d'amidinium à 1625 $cm^{-1}$. On élimine alors l'excès de chlorure d'oxalyle par dégazage à l'aide d'un courant d'azote sec et la solution obtenue est utilisée telle quelle pour la condensation ultérieure avec la diamine (étape 3).

ETAPE 3.

Polycondensation du sel de diformamidinium avec la N,N-diméthylhexane diamine-1,6.

A la solution précédente, on additionne lentement une solution de 2,8 ml ( 2,2 g ; 0,0153 mole) de diamine dans 20 ml de dichloroéthane sec, sous atmosphère sèche . La température se maintient aux alentours de 40°C au cours de l'addition . On laisse ensuite réagir en agitant pendant 18 heures à température ambiante . On filtre le précipité de chlorhydrate de triéthylamine et on concentre le filtrat au 1/2 et on extrait par 3 fois 50 ml d'eau bipermutée . On concentre à sec la phase aqueuse, reprend au dichlorométhane, sèche sur sulfate de sodium et concentre à sec .On lave à l'acétate d'éthyle chaud puis dissout dans de l'acétonitrile anhydre et on concentre à sec sous bon vide.

On a obtenu 8,9 g ( rendement 90%) d'un produit très visqueux de couleur marron clair.

I.R.T.F._Bandes caractéristiques du guanidinium à 1549 et 1567 $cm^{-1}$ . Disparition de la bande amidinium à 1625 $cm^{-1}$.

R.M.N. $^1$H 250 MHz (acétone $D_6$, TMS); δ ppm 0,95 (t,J=7,1 Hz 6H);1,25-1,55 (m,8H); 1,55-1,95 (m,8H); 3,06-3,27 (m,6H); 3,27-3,75 (m,8H).

Soluble dans :l'acétone, le dichloroéthane, le dichlorométhane, l'acétonitrile et l'eau.

Insoluble dans : le benzène, le chloroforme et l'acétate d'éthyle.

Etape 4:

Préparation du silane polycondensat.

A une solution de 7,90 g du polycondensat de l'étape 3 dans 40 ml de 1,2 dichloroéthane sec on ajoute une solution de 0,7 g ($3.10^{-3}$ mole) de N-butylamino-(propyl-triméthoxysilane) et de 1,2 ml ($8.10^{-3}$ mole) de triéthylamine dans 5 ml de 1,2 dichloroéthane sec .

Le mélange est porté au reflux pendant quelques heures . Après refroidissement 1,1 g (3,10-3 mole) de TBCA en solution dans 10 ml de 1,2 dichloroéthane sec sont additionnés. Le milieu réactionnel est alors laissé sous agitation à température ambiante pendant 2 heures .

Après filtration du chlorhydrate de triéthylamine, la phase organique est concentrée et lavée par 20 ml d'éther de pétrole (45-60°C) . On obtient alors 8,5 g de polymère liquide .

Analyses .

I.R.T.F._bandes caractéristiques du guanidinium 1540-1550$cm^{-1}$-RMN 80 MHz : δppm : 0,6 (t; 2H; $CH_2$-Si-); 0,95 (t; 30H; $CH_3$-$CH_2$-); 1,5 (m;70H;-$CH_2$-$CH_2$-); 3(s;18H; $CH_3N^+$-); 3,2 (m;36H;$CH_2$-N-+) ; 3,5 (s; 9H; $CH_3$O-Si).

Etape 5 .

Greffage du silane-polycondensat sur silice.

A 7g de silice (90$m^2$/g; 4,8 µmol/$m^2$) dans 50 ml de 1,2-dichloroéthane sec on ajoute une solution de 5,73g (4,4 mmole) de silane-polycondensat de l'étape 4 dans 10 ml de 1,2-dichloroéthane sec. Le mélange est porté au reflux pendant 24 heures.

Après refroidissement la silice est filtrée et lavée au soxhlet pendant 3 heures au dichlorométhane puis séchée quelques heures à 50°C sous 2,6 kPa (20 mm Hg) .

Analyses .

I.R.T.F._(réflexion diffuse) : bande guanidinium à 1540 $cm^{-1}$
Analyse élémentaire .
Taux de chlore : 0,60% soit 0,2 meq/g

Etape 6.

Blocage des fonctions silanols résiduelles.

Le blocage est réalisé dans des conditions identiques à celles utilisées dans l'exemple 1 , étape 4.

Analyses.

I.R.T.F. (réflexion diffuse): bande guanidinium à 1540 $cm^{-1}$
Taux de chlore : 0,58 %

EXEMPLE 3 :

Evaluation de l'activité catalytique du support à base de silice de formule (I) , préparé à l'exemple 1, dans la phosgénation de l'acide éthyl-2 hexanoïque.

On introduit dans un réacteur , 1 eq. d'acide éthyl-2 hexanoïque et la quantité correspondant à 0,01 eq de motif guanidinium de silice greffée. On porte le milieu réactionnel à 120,5°C et on envoie le phosgène gazeux. L'avancement de la réaction est suivi par chromatographie en phase gazeuse .

Après dégazage sous courant d'argon, on sépare la silice par filtration et on lave 3 fois avec 20 ml (pour 3,5 g de silice) de chloroforme . Après lavage au dichlorométhane au soxhlet pendant 2 heures, la silice est séchée à 80°C sous 2,6 kPa ( 20 mm Hg ) pendant 18 heures et analysée.

Deux autres opérations de phosgénation sont réalisées de la même manière , y compris le traitement du catalyseur, à partir de la silice récupérée de l'opération précédente .

Les quantités de matières engagées et les résultats obtenus sont rassemblés dans les tableaux I et II ci-après.

Il est à noter qu'aucune perte de poids de la silice n'est observée entre les différentes opérations.

EXEMPLE 4 :

Evaluation de l'activité catalytique du support à base de silice de formule [I] après traitement prolongé en milieu phosgène et chlorure d'acide.

Cet exemple est destiné à montrer la très grande stabilité des catalyseurs selon l'invention.

On place 10 g du support de formule [I] préparé à l'exemple 1 dans 100 ml de chlorure d'éthyl-2 hexanoyle sous balayage de phosgène à 120°C pendant 64 heures . A l'issue de ce traitement , on ne constate pas d'évolution, au niveau du spectre IR, du taux de chlore et du résultat de l'analyse élémentaire .
Chlore % : 0,59; C% : 3,14 ; H% : 0,82; N%: 0,33.

On répète ensuite une opération de phosgénation de l'acide éthyl-2 hexanoïque dans les mêmes conditions de l'exemple 3 avec

- 3,5 g (0,7 m.eq) du catalyseur ayant subi le traitement ci-dessus (120°C-64 h )
- 11,6 g (0,08 mole) d'acide éthyl-2 hexanoïque.

La phosgénation dure 7 h et la composition du produit obtenu est la suivante:

| | |
|---|---|
| - teneur en chlorure d'éthyl-2 hexanoyle | 99,9% |
| - teneur en acide éthyl-2 hexanoïque | 0 |
| - teneur en anhydride | 0,1%. |

EXEMPLE 5 :

Evaluation de l'activité catalytique de la silice non greffée utilisée comme matière première du support à base de silice de formule [I] de l'exemple 1.

On répète l'opération de phosgénation de l'exemple 4 mais avec de la silice brute (90 $m^2$/g; 4,8 µmole/$m^2$) non fonctionnalisée sur les quantités suivantes :

| | |
|---|---|
| - silice | 3,5 g |
| - acide éthyl-2 hexanoïque | 11,6 g. |

On arrete l'opération après 8h de phosgénation à 122°C. On constate que la teneur en chlorure d'acide n'est que de 18%.

EXEMPLE 6 :

Evaluation de l'activité catalytique du support de formule (I) préparé à l'exemple 1 dans la phosgénation de l'acide stéarique .

On opère comme à l'exemple 3 , la phosgénation étant réalisée à 100°C au lieu de 120,5°C , avec :

- 10 g ( 2 m eq. $Cl^-$ ) du support de formule (I) préparé à l'exemple 1,
- 56,9 g ( 0,2 mole) d'acide stéarique .

La réaction est terminée après 6 h de phosgénation . La composition du produit obtenu est la suivante :

| | |
|---|---|
| - teneur en chlorure de stéaroyle | 99,7 % |
| - teneur en acide stéarique | 0 |
| - teneur d'anhydride | 0,3 % |

EXEMPLE 7 :

Préparation du chlorure d'éthyl-2 hexanoyle en continu sur colonne du support de formule (I) préparé à l'exemple 1.

On alimente à contre-courant une colonne à double enveloppe chauffée à 120°C et remplie du support à base de silice de formule (I) préparé à l'exemple 1.

- à la partie supérieure par de l'acide éthyl-2-hexanoïque ,
- à la partie inférieure par du phosgène gazeux.

Le rapport des débits exprimés en moles/heure $COCl_2$/acide est de 1,1.

Les débits sont choisis de façon que le temps de contact dans la colonne soit de 4 heures.

Le chlorure d'acide, recueilli en bas de colonne et dégazé (élimination du phosgène dissous), possède la composition suivante :

| | |
|---|---|
| - teneur en chlorure d'éthyl-2 hexanoyle | 99,7 % |
| - teneur en acide éthyl-2 hexanoïque | 0 |
| - teneur en anhydride | 0,3%. |

Les supports à base de silice, modifiés par greffage de groupements polyalkylguanidinium , selon l'invention , sont des catalyseurs de phosgénation de valeur pour la transformation d'acides carboxyliques en chlorures d'acides . Ils permettent d'atteindre d'excellents rendements en étant stables et aisément recyclables . En raison de leurs propriétés avantageuses , ils peuvent être utilisés non seulement dans des réactions de phosgénation avec des acides carboxyliques connus et déjà mis en oeuvre dans ce type de réaction, mais aussi pour l'obtention de chlorures d'acides qu'il était très difficile d'obtenir par phosgénation avec les catalyseurs actuellement disponibles.

EXEMPLES 8 à 10:

Préparation des chlorures de trichloroacétyle, de p-nitrobenzoyle et de 3,4,5-triméthoxybenzoyle au moyen du diphosgène.

On utilise le mode opératoire général suivant:

Dans un réacteur équipé avec un réfrigérant à chlorure de méthylène et carboglace, on introduit une solution de l'acide carboxylique (12,2 mmol) dans 20 ml de chlorobenzène contenant 0,7 g (0,122 meq $Cl^-$) du catalyseur de même formule qu'à l'exemple I (appelé ci-après PBGSiCl) mais préparé à partir d'une silice possédant un taux d'hydroxyle légèrement plus faible, et qui a donc un taux de chlore de 0,17 meq/g.

La suspension est chauffée à 130°C et on ajoute goutte à goutte 1,5 ml (12,2 mmol) de chloroformiate de trichlorométhyle (diphosgène). La décomposition est instantanée et on obtient un bon reflux de phosgène. Lorsque le dégagement d'acide chlorhydrique est terminé (4-9 heures) on arrête la réaction. On sépare le catalyseur par filtration, on évapore le solvant et on purifie le résidu obtenu par distillation.

Les conditions particulières, les rendements et les caractéristiques des chlorures d'acides obtenus sont rassemblés dans le tableau 3 ci-après.

EXEMPLES 11 à 14:

Préparation des chlorures de trichloroacétyle, de trifluoroacétyle, de p-nitrobenzoyle et de 3,4,5-triméthoxybenzoyle au moyen du chlorure de thionyle.

On utilise le mode opératoire général suivant :

On ajoute 3 ml (40 mmol) de chlorure de thionyle fraîchement distillé dans 17,4 mmol d'acide carboxylique et 0,85 g (0,17 meq $Cl^-$ ) du catalyseur PBGSiCl. On chauffe à 80°C le mélange réactionnel et on maintient la température jusqu'à la disparition complète de la bande IR caractéristique de l'acide carboxylique (1-8 heures). On arrête la réaction, on sépare le catalyseur par filtration et on évapore le chlorure de thionyle sous pression réduite. On distille ensuite pour obtenir le produit souhaité.

Les conditions particulières et les rendements obtenus pour chacun des chlorures sont rassemblés dans le tableau 4 ci-après.

EXEMPLES 15 à 23 : Préparation des esters.

On utilise le mode opératoire général suivant.

On ajoute 0,017 mol de chloroformiate à une suspension agitée de 1 g ( 0,17 meq $Cl^-$) du catalyseur PBGSiCl dans 0,017 mol de l'acide carboxylique chauffée à 120°C . Le mélange réactionnel est agité à la même température pendant 2 à 9 h jusqu'à la fin du dégagement de $CO_2$ et de HCl. On le dilue ensuite avec du dichlorométhane anhydre et on élimine le catalyseur par filtration.On évapore le dichlorométhane et on obtient l'ester par distillation.

Les résultats pour chaque exemple sont rassemblés dans le tableau 5 ci-après.

Les caractéristiques des esters obtenus sont les suivantes :

2-éthyl hexanoate de phényle

F:50°-51°C; IRTF(KBr):1745$cm^{-1}$ ; RMN $^1H$ ($CDCl_3$;TMS) δppm:2,1-2,2(s;9H);6,75 (m;2H).

Caprylate d'éthyle

Eb:40°C/0,2 mmHg; IRTF(film):1740 $cm^{-1}$; RMN $^1H$ ($CDCl_3$;TMS)δppm: 0,7-2,0 (m,16H); 2,3 (t, 1H); 4,3 (q,2H);

Caprylate de 2,2,2-trichloroéthyle

bp:50°C/0,2 mmHg,IRTF(film): 1760 $cm^{-1}$ ; RMN $^1H$($CDCl_3$; TMS) δppm:0,7-2,1(m,16H); 2,5(t, 2H); 4,7 (s,2H).

Benzoate de phényle

F:68-70°C; IRTF(film):1730 $cm^{-1}$;RMN $^1H$ ($CDCl_3$; TMS); tous les protons ont des signaux entre 6,7 et 7,1 ppm.

Benzoate de 2,2,2-trichloroéthyle

Eb:110°C/0,03 mmHg; IRTF(film):1740$cm^{-1}$;RMN $^1H$ ($CDCl_3$;TMS) δppm:5,0(s,2H); 7,5 (m,3H); 8,25 (m;2H).

Mésitoate de phényle

F: 50°C; IRTF(film); 1745 $cm^{-1}$;RMN $^1H$ ($CDCl_3$; TMS) δppm: 2,1-2,2(s, 9H); 6,75 (m,2H); 7,25 (m,5H).

Mésitoate de benzyle

F:165°C; (IRTF (KBr): 1724 $cm^{-1}$; RMN $^1H$ ($CDCl_3$; TMS) δppm:2,1-2,2 (s,9H): 5,1(s,2H); 6,6 (s,2H); 7,2(m,5H)

Pivalate de phényle

Eb: 55°C/1mmHg; IRTF(film):1750 $cm^{-1}$; RMN $^1H$ ($CDCl_3$;TMS) δppm: 1,2(s; 9H); 7,2(m,5H)

Pivalate de benzyle

Eb:62°C /2mmHg ; IRTF(film):1732 $cm^{-1}$; RMN $^1H$ ($CDCl_3$;TMS)δppm:1,2 (s,9H); 5,1(s,2H); 7,2 (m,5H).

TABLEAU I

Conditions opératoires des réactions de phosgénation

| N° op. | OPERATION | Quantité de silice | Quantité d'acide | Quantité de $COCl_2$ | Durée | Composition du chlorure d'acide obtenu | | |
|---|---|---|---|---|---|---|---|---|
| | | | | | | teneur en chlorure. d'acide % | teneur en acide % | teneur en anhydride % |
| 1 | 1ère phosgénation | 3,5 g (0,7meq) | 10 g (0,07 mole) | 19,5 g | 7 h | 99 | 0,08 | 0,92 |
| 2 | 2ème phosgénation (1er recyclage) | 3,4 g | 9,8 g | 18,9 g | 7 h15 mm | 99,6 | 0,05 | 0,35 |
| 3 | 3ème phosgénation (2ème recyclage ) | 3,2 | 9,5g | 21,6 | 8 h | 99,3 | 0 | 0,7 |

TABLEAU II

| Analyse élémentaire de la silice après chaque opération | | | | | |
|---|---|---|---|---|---|
| N° | ANALYSES | C% | H% | N% | Cl% |
| 1 | 1ère phosgénation | 3,31 | 0,82 | 0,38 | 0,49 |
| 2 | 2ème phosgénation | 3,14 | 0,81 | 0,43 | 0,49 |
| 3 | 3ème phosgénation | 3,20 | 0,78 | 0,39 | 0,57 |

TABLEAU 3

| Exemple | Acide | Temps (heure) | Chlorure d'acide rendement [a] % | Point d'ébullition (Eb) ou de fusion(F) °C | IRTF $cm^{-1}$ | RMN $^1H$ ($CDCl_3$) δppm |
|---|---|---|---|---|---|---|
| 8 | trichloro-acétique | 9 | 92 | Eb : 115 | film 1805 | |
| 9 | 3,4,5-triméthoxy benzoïque | 6 | 90 | Eb:90/0,1 mmHg | (KBr) 1754 | 3,9(s,9H) 7,4 (s,2H) |
| 10 | p-nitrobenzoïque | 4 | 85 | F : 72 | (KBr) 1779 | protons 8,2 -8,5 |

[a] : calculé à partir des composés isolés

TABLEAU 4

| Exemple | Acide | Temps (heure) | Clorure d'acide: rendement % |
|---|---|---|---|
| 11 | trichloroacétique | 5 | 90[a] |
| 12 | trifluoroacétique | 7,5 | 70[b] |
| 13 | 3,4,5-triméthoxybenzoïque | 2 | 90[a] |
| 14 | p-nitrobenzoïque | 1 | 94[a] |

[a]: Calculé à partir des composés isolés.
[b]: Le chlorure de trifluoroacétyle gazeux a été piégé par la N,N-dibutylamine et a été caractérisé et son rendement calculé sous la forme N,N-dibutyl trifluoroacétamide. Eb : 60°C/0,05 mmHg; IRTF (film) : 1691 $cm^{-1}$; RMN $^1H$ ( $CDCl_3$ , δppm): 0,9 ( t, 6 H); 1-1,6 (m,8H); 3,35 ( t, 4H).

TABLEAU 5

| Ex. | Acides (RCOOH) R | Chloroformiates ( R'OCOCl) R': | Temps (heures) | T°C | Rendement (a) % RCOOR'(autres) | Rendement([b]) Lit. |
|---|---|---|---|---|---|---|
| 15 | $CH_3(CH_2)_3CH(C_2H_5)-$ | C6H5 | 9 | 120 | 98 (<1) | - |
| 16 | $CH_3(CH_2)_6$ - | $C_2H_5$ | 4 | 120 | 95(<1) | |
| 17 | $CH_3(CH_2)_6$ - | $CH_2CCl_3$ | 2 | 120 | 83(<1) | 90(10) [c] |
| 18 | $C_6H_5$ - | $C_6H_5$ | 5 | 120 | 92(<1) | - |
| 19 | $C_6H_5$ - | $CH_2CCl_3$ | 3 | 120 | 84(<1) | 20(80) [c] |
| 20 | 2,4,6 | $C_6H_5$ | 8 | 120 | 97(<1) | - |
| 21 | $(CH_3)_3C_6H_2$ - | $CH_2C_6H_5$ | 8 | 120 | 96(<1) | 0(98) [d] |
| 22 | $(CH_3)_3C$ - | $C_6H_5$ | 9 | 120 | 83(<1) | - |
| 23 | $(CH_3)_3C$ - | $CH_2C_6H_5$ | 8 | 120 | 95(<1) | 47(52) [e] |

a : Rendement calculé à partir des produits isolés pour les esters; pour les autres produits entre parenthèses rendement déterminé par RMN $^1H$
b: Selon la publication de Kim S. et al, J. Org. Chem. 1985, 50, 560 avec 10 à 50% de 4-(diméthylamino)pyridine dans $CH_2Cl_2$ , 0-25°C 0,3-1 heure , en présence de triéthylamine en quantité stoechiométrique.
c: rapport ester: carbonate
d: 98% d'anhydride
e: 52% d'anhydride.

**Revendications**

1. Support à base de silice , modifié en surface par greffage par liaisons covalentes de groupements organiques , caractérisé en ce que lesdits groupements sont des groupements guanidinium polysubstitués et de préférence des groupements polyalkylguanidinium.

2. Support selon la revendication 1, caractérisé en ce qu'il répond à la formule (I) suivante :

(I)

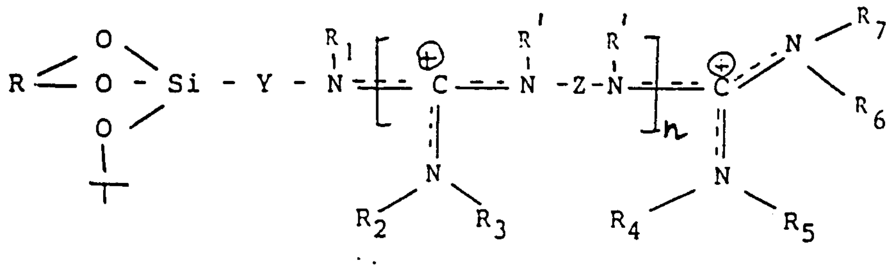

, $(n+1)A^{\ominus}$

dans laquelle :

- R représente le support à base de silice
- Y représente un radical alkyle bivalent ramifié ou non de $C_2$ à $C_{10}$ comportant au moins 2 atomes de carbone sur la chaîne linéaire entre les atomes de silicium et d'azote ,
- Z représente un radical aliphatique bivalent de $C_2$ à $C_{10}$,
- R' représente un radical alkyle de $C_1$ à $C_4$,
- $R_1$, $R_2$, $R_3$, $R_4$, $R_5$, $R_6$, $R_7$, identiques ou différents représentent des radicaux alkyles de $C_1$ à $C_6$,
- A représente un anion possédant une seule charge négative, et
- n = 0 ou n est un nombre entier.
- T représente
  - * une liaison simple avec le support R
  - * un groupe $-Si(R_o)_3$ dans lequel $R_o$ est un radical alkyle en $C_1$ à $C_4$ , préférentiellement un radical méthyle, ou
  - * un groupe alkyle en $C_1$ à $C_4$

3. Support selon la revendication 2 ,caractérisé en ce que Y représente un radical $-(CH_2)_3-$.

4. Support selon l'une des revendications 2 ou 3 , caractérisé en ce que $R_1$ à $R_7$ représentent des radicaux méthyle et/ou n-butyle.

5. Support selon l'une des revendications 2 à 4, caractérisé en ce que A représente un ion halogénure et en particulier un ion chlorure ou un ion $HCl_2^-$.

6. Support selon l'une des revendications 1 à 5, caractérisé en ce que les fonctions silanols résiduelles ROH sont sous la forme $ROSi(CH_3)_3$.

7. Ion triméthoxysilane polyalkylguanidinium répondant à la formule (II) suivante :

(II)

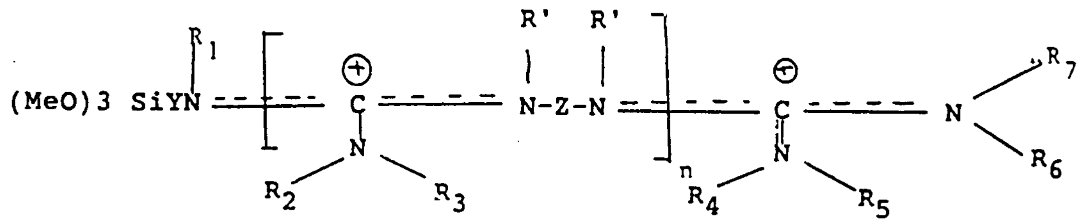

dans laquelle n , Y, Z , R' et $R_1$ à $R_7$ ont la même signification que dans la revendication 2 et Me représente le radical méthyle.

8. Procédé de préparation des supports selon l'une des revendications 1 à 6 par réaction de la silice avec un ion polyalkylguanidinium .

9. Procédé selon la revendication 8 , caractérisé en ce que l'ion polyalkylguanidinium est un ion selon la revendication 7 .

10. Procédé selon la revendication 9 , caractérisé en ce que lorsque n = 0 , l'ion polyalkylguanidinium selon la revendication 7 est obtenu par réaction d'une amine primaire avec un halogénure de triméthoxysilylalkyle puis par réaction de la triméthoxysilylamine secondaire obtenue avec un sel de formamidinium.

11. Procédé selon la revendication 9 , caractérisé en ce que lorsque n est différent de 0 , l'ion polyalkyl-guanidinium selon la revendication 7 est obtenu par polycondensation d'un sel de diformamidinium avec une diamine puis par réaction du polyguanidinium ainsi obtenu avec une triméthoxysilylalkylamine, puis par réaction du polymère obtenu avec un sel de formamidinium.

12. Procédé selon l'une des revendications 8 à 11, caractérisé en ce que l'on traite ensuite le support obtenu avec de l'hexaméthyldisilazane.

13. Utilisation des supports selon l'une des revendications 1 à 6 pour la catalyse de la chloruration des acides carboxyliques en chlorures d'acide .

14. Utilisation des supports selon l'une des revendications 1 à 6 pour la catalyse de l'estérification des acides carboxyliques au moyen des chloroformiates.

## Claims

1. Silica-based substrate, surface modified by grafting,via covalent bonds, organic groups, characterized in that said groups are polysubstituted guanidinium groups, preferably polyalkylguanidinium groups.

2. Substrate according to claim 1 characterized in that it has the following formula (I):

(I)

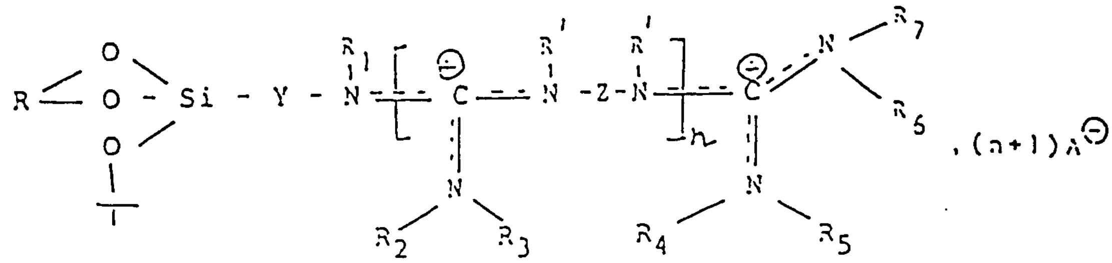

wherein:

- R represents the silica-based substrate,
- Y represents a $C_2$-$C_{10}$ bivalent alkyl radical, branched or not, having at least 2 carbon atoms on the linear chain between silicon and nitrogen atoms,
- Z represents a $C_2$-$C_{10}$ bivalent aliphatic radical,
- R' represents a $C_1$-$C_4$ alkyl radical,
- $R_1$, $R_2$, $R_3$, $R_4$, $R_5$, $R_6$, $R_7$, identical or different, represent $C_1$-$C_6$ alkyl radicals,
- A represents an anion having only one negative charge, and
- n = 0 or n is an integer,
- T represents :
    - * a single bond with the R substrate
    - * a group - $Si(R_o)_3$ wherein $R_o$ is a $C_1$-$C_4$ alkyl group, preferably a methyl radical, or
    - * a $C_1$-$C_4$ alkyl group.

3. Substrate according to claim 2 characterized in that Y represents a $-(CH_2)_3-$radical.

4. Substrate according to any of claims 2 or 3 characterized in that $R_1$ to $R_7$ represent methyl and/or n-butyl radicals.

5. Substrate according to any of claims 2 to 4 characterized in that A represents an halogenide ion, particularly a chloride ion or a $HCl2^-$ ion.

6. Substrate according to any of claims 1 to 5 characterized in that the remaining silanol functions ROH are under the form $ROSi(CH_3)_3$.

7. Trimethoxysilane polyalkylguanidinium ion of following formula (II):

(II)

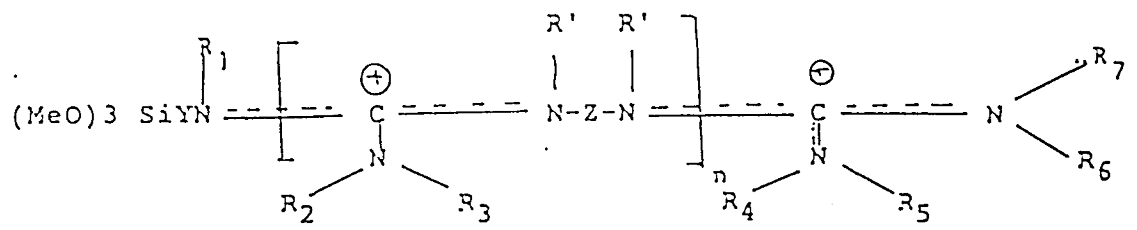

wherein n, Y, Z, R' and $R_1$ to $R_7$ have the same meanings as in claim 2 and Me represents the methyl radical.

8. Process for preparing substrates according to any of claims 1 to 6 by reaction of silica with a polyalkylguanidinium ion.

9. Process according to claim 8 characterized in that the polyalkylguanidinium ion is an ion according to claim 7.

10. Process according to claim 9 characterized in that when n = 0 the polyalkylguanidinium, ion according to claim 7 is obtained by reaction of a primary amine with a trimethoxysilylalkyl halogenide, and then by reaction of the resulting secondary trimethoxysilylamine with a formamidinium salt.

11. Process according to claim 9 characterized in that when n is different from 0 the polyalkylguanidinium ion according to claim 7 is obtained by polycondensation of a diformamidinium salt with a diamine and then by reaction of the resulting polyguanidinium with a trimethoxysilylalkylamine and finally by reaction of the resulting polymer with a formamidinium salt.

12. Process according to any of claims 8 to 11 characterized in that the resulting substrate is then treated with hexamethyldisilazane.

13. Use of substrates according to any of claims 1 to 6 as a catalyst for chlorination of carboxylic acids into acid chlorides.

14. Use of substrates according to any of claims 1 to 6 as a catalyst for esterification of carboxylic acids with chloroformates.

**Patentansprüche**

1. Träger auf Siliciumoxidbasis, der an der Oberfläche durch über kovalente Bindungen aufgepfropfte organische Gruppen modifiziert ist, dadurch gekennzeichnet, daß es sich bei den Gruppen um polysubstituierte Guanidiniumgruppen und vorzugsweise um Polyalkylguanidiniumgruppen handelt.

2. Träger gemäß Anspruch 1, dadurch gekennzeichnet, daß er der folgenden Formel (I) entspricht:

(I)

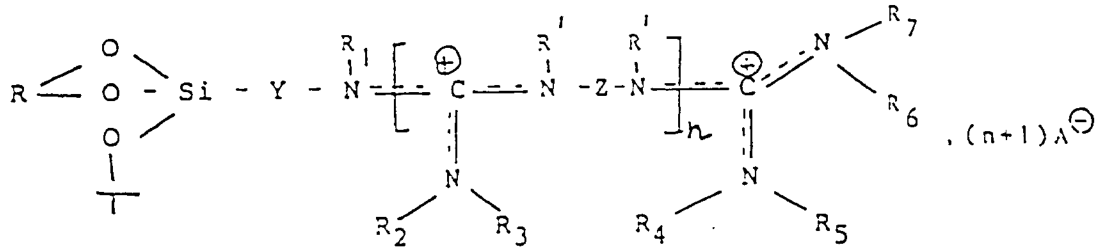

in der:

- R den Träger auf Siliciumoxidbasis darstellt;
- Y einen zweiwertigen verzweigten oder nichtverzweigten $C_2$- bis $C_{10}$-Alkylrest darstellt, der auf der linearen Kette zwischen dem Silicium- und dem Stickstoffatom wenigstens 2 Kohlenstoffatome trägt;
- Z einen zweiwertigen aliphatischen $C_2$- bis $C_{10}$-Rest darstellt;
- R' einen $C_1$- bis $C_4$-Alkylrest darstellt;
- $R_1$, $R_2$, $R_3$, $R_4$, $R_5$, $R_6$ und $R_7$ gleich oder verschieden sind und $C_1$- bis $C_6$-Alkylreste darstellen;
- A ein Anion mit einer einfachen negativen Ladung darstellt und
- n = 0 oder n eine ganze Zahl ist;
- T
  * eine Einfachbindung mit dem Träger R,
  * eine $Si(R_o)_3$-Gruppe, in der $R_o$ ein $C_1$- bis $C_4$-Alkylrest, vorzugsweise ein Methylrest, ist, oder
  * eine $C_1$- bis $C_4$-Alkylgruppe darstellt.

3. Träger gemäß Anspruch 2, dadurch gekennzeichnet, daß Y einen $-(CH_2)_3$-Rest darstellt.

4. Träger gemäß einem der Ansprüche 2 oder 3, dadurch gekennzeichnet, daß $R_1$ bis $R_7$ Methyl- und/oder n-Butylreste darstellen.

5. Träger gemäß einem der Ansprüche 2 bis 4, dadurch gekennzeichnet, daß A ein Halogenidion und insbesondere ein Chloridion oder ein $HCl_2^-$-Ion darstellt.

6. Träger gemäß einem der Ansprüche 1 bis 5, dadurch gekennzeichnet, daß die restlichen Silanolfunktionen ROH in der Form $ROSi(CH_3)_3$ vorliegen.

7. Polyalkylguanidiniumtrimethoxysilanion, das der folgenden Formel (II) entspricht:

(II)

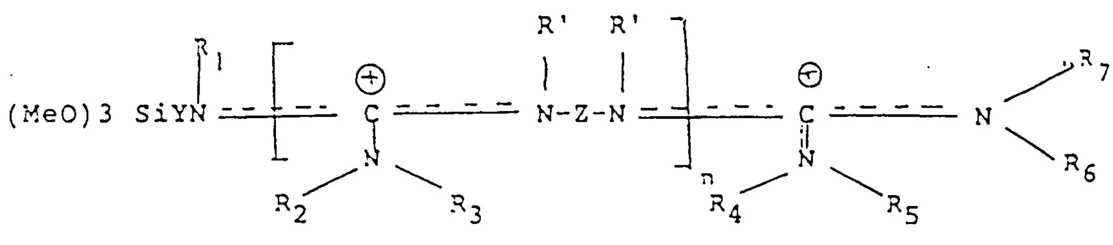

in der n, Y, Z, R' und $R_1$ bis $R_7$ dasselbe wie in Anspruch 2 bedeuten und Me den Methylrest darstellt.

8. Verfahren zur Herstellung von Trägern gemäß einem der Ansprüche 1 bis 6 durch Reaktion von Siliciumoxid mit einem Polyalkylguanidiniumion.

9. Verfahren gemäß Anspruch 8, dadurch gekennzeichnet, daß es sich bei dem Polyalkylguanidiniumion um ein Ion gemäß Anspruch 7 handelt.

10. Verfahren gemäß Anspruch 9, dadurch gekennzeichnet, daß das Polyalkylguanidiniumion gemäß Anspruch 7, wenn n = 0 ist, durch Reaktion eines primären Amins mit einem Trimethoxysilylalkylhalogenid und anschließende Reaktion des erhaltenen sekundären Trimethoxysilylamins mit einem Formamidiniumsalz erhalten wird.

11. Verfahren gemäß Anspruch 9, dadurch gekennzeichnet, daß das Polyalkylguanidiniumion gemäß Anspruch 7, wenn n von 0 verschieden ist, durch Polykondensation eines Diformamidiniumsalzes mit einem Diamin und anschließende Reaktion des so erhaltenen Polyguanidiniums mit einem Trimethoxysilylalkylamin und anschließende Reaktion des erhaltenen Polymers mit einem Formamidiniumsalz erhalten wird.

12. Verfahren gemäß einem der Ansprüche 8 bis 11, dadurch gekennzeichnet, daß man den erhaltenen Träger anschließend mit Hexamethyldisilazan behandelt.

13. Verwendung der Träger gemäß einem der Ansprüche 1 bis 6 für die Katalyse der Chlorierung von Carbonsäuren zu Säurechloriden.

14. Verwendung der Träger gemäß einem der Ansprüche 1 bis 6 für die Katalyse der Veresterung von Carbonsäuren mittels Chlorformiaten.